Europäisches Patentamt

European Patent Office

Office européen des brevets

(11) Numéro de publication: **0 514 576 A1**

# DEMANDE DE BREVET EUROPEEN

(21) Numéro de dépôt: **91108412.7**

(22) Date de dépôt: **24.05.91**

(51) Int. Cl.5: **A61K 7/00,** A23D 9/06,
C11B 5/00

(43) Date de publication de la demande:
**25.11.92 Bulletin 92/48**

(84) Etats contractants désignés:
**AT BE CH DE DK ES FR GB GR IT LI LU NL SE**

(71) Demandeur: **SOCIETE DES PRODUITS NESTLE S.A.**
**Case postale 353**
**CH-1800 Vevey(CH)**

(72) Inventeur: **Colarow, Ladislas**
**Eden-Roc 12**
**CH-1073 Savigny(CH)**

(54) **Mélange antioxydant liposoluble.**

(57) Pour protéger une matière grasse sensible à l'oxydation ou un produit alimentaire ou cosmétique contenant une matière grasse sensible à l'oxydation, on y incorpore un mélange antioxydant constitué d'un complexe de vitamine du groupe B et d'un acide organique, notamment l'ascorbate de niacinamide et d'une lécithine riche en phosphatidylcholine.

Le mélange permet en particulier de mettre la vitamine C sous forme liposoluble.

L'invention concerne un mélange antioxydant liposoluble à base d'acide organique.

L'intérêt qu'il y a à présenter les acides organiques antioxydants sous une forme liposoluble est dû au fait qu'ils exercent une activité inhibitrice de l'oxydation des lipides dans les systèmes biologiques. En combinaison avec la vitamine E, la vitamine C par exemple exerce une acitvité antioxydante synergique car, en tant que réducteur, l'acide L-ascorbique transforme les dérivés tocophéroxyles en tocophérols qui peuvent à nouveau fixer les radicaux libres sous forme de dérivés tocophéroxyles.

L'acide L-ascorbique, hydrophile, peut être rendu liposoluble par estérification de ses groupes hydroxyles en position 5 ou 6 avec un acide gras. Ainsi, l'acide 6-0-palmitoyl-L-ascorbique ou ascorbyl-palmitate est utilisé comme additif pour protéger les matières grasses de l'oxydation et également comme source de vitamine C. Ce composé présente cependant l'inconvénient de se dissoudre extrêmement lentement dans les matières grasses aux températures auxquelles il ou elles ne se dégradent pas. On a ainsi recours a l'addition de glycérides partiels ou de lipides complexes, par exemple de lécithine de soja. Mais même avec ces additifs, l'incorporation de palmitate d'ascorbyle à un niveau assurant un pouvoir antioxydant suffisant ne peut s'effectuer qu'à température relativement élevée, de l'ordre de 130°C pendant une certaine durée, par exemple 30 min. ce qui induit une dégradation des matières grasses comme de l'antioxydant lui-même dont le point de fusion est 115,5°C.

On a proposé, par exemple selon la demande de brevet EP-A-0326829 de réaliser un mélange antioxydant liposoluble solide à température ambiante contenant l'acide ascorbique à base de lipides polaires tels que les lécithines. Un tel mélange peut être utilisé sans inconvénient dans les produits solides. Cependant, son incorporation dans les huiles ne peut être réalisée qu'en chauffant à environ 120-130°C, ce qui n'est pas envisageable dans le cas des huiles très sensibles à la chaleur, comme par exemple les huiles végétales ou de poisson riches en acides gras insaturés. A l'entreposage, par exemple, l'huile de pépin de cassis contenant en particulier un mélange d'acide ascorbique, de lécithine et de tocophérol se sépare avec le temps en deux phases par décantation du mélange antioxydant. Par ailleurs, lorsque l'on encapsule une telle huile stabilisée dans la gélatine, l'acide ascorbique en présence de l'eau à la surface de la gélatine entraîne à la longue un dégommage de la lécithine se traduisant par la formation d'un précipité.

Par ailleurs, selon US-A-2.432.698 par exemple, les vitamines A et D peuvent être protégées de l'oxydation par addition de niacinamide. Lorsqu'il s'agit de stabiliser ces vitamines avec comme support une huile, le cas échéant en présence de tocophérol, la niacinamide n'étant pas liposoluble, on a recours selon ce brevet à l'addition au mélange de solvants naturels comme par exemple les alcools éthylique et laurique.

L'invention se propose de résoudre le problème de l'incorporation d'acides organiques notamment de l'acide ascorbique dans un mélange antioxydant liposoluble constitué d'ingrédients naturels ne présentant pas les inconvénients précités.

Le mélange antioxydant selon l'invention est caractérisé par le fait qu'il est constitué d'un complexe de vitamine du groupe B et d'acide organique et d'une lécithine contenant au moins 40% en poids de phosphatidylcholine.

Parmi les vitamines du groupe B, on préfère les bases pyridiniques comme par exemple la niacine, la niacinamide et la pyridoxyne.

Par acide organique selon l'invention, on entend un polyacide capable de former un complexe avec une base vitaminique du groupe B, tel que par l'exemple l'acide citrique, tartrique ou de préférence l'acide ascorbique.

Dans le contexte de l'invention, la lécithine utilisée est de préférence une fraction de lécithine de soja soluble dans l'alcool éthylique. Celle-ci est zwitterionique c'est-à-dire stable dans une gamme large de pH et contient peu de phospholipides complexables. La lécithine peut se présenter de préférence sous forme d'un mélange contenant environ 40-50% en poids de phosphatidylcholine et environ 50% en poids d'huile végétale, par exemple de soja, de triglycéride à chaîne moyenne (triglycéride d'acides gras en C8-C10) ou de beurre de cacao.

Pour préparer le mélange antioxydant liposoluble, on dissout le complexe, par exemple l'ascorbate de niacinamide, de préférence dans un solvant de qualité alimentaire, par exemple l'éthanol ayant été de préférence préalablement dégasé. Le complexe lui-même peut être préparé, par exemple par chauffage modéré d'un mélange équimolaire de niacinamide et d'acide ascorbique dans un solvant polaire approprié, par exemple l'alcool éthylique, l'acétone ou l'eau. La dissolution du complexe peut s'effectuer par agitation modérée à température de 40 -50°C, de préférence sous atmosphère inerte, par exemple d'azote, à une concentration de 2 à 10 g de complexe pour 100 ml de solvant. On y ajoute ensuite le cas échéant le d,l-alphatocophérol. 5-10 volumes de cette solution sont alors mélangés à 1-2 volumes de lécithine qui se dissout rapidement sous agitation modérée à 50-60°C, de préférence sous atmosphère inerte. On peut ensuite concentrer la solution en évacuant le solvant, par exemple par distillation sous gaz inerte à 60-

2

65°C, puis éliminer toute trace de solvant résiduel, par exemple par barbottage d'un gaz inerte à la température ambiante. Le mélange obtenu se présente sous forme d'une solution translucide de couleur jaune clair semblable à une huile végétale raffinée ayant la viscosité d'une lécithine de soja standard du commerce, c'est-à-dire contenant environ 35-40% en poids d'huile de soja. Il a une coloration ambrée, est translucide, inodore et stable à la chaleur.

Le mélange antioxydant peut être entreposé à l'abri de la lumière et à température ambiante.

Il peut être utilisé tel quel et être facilement incorporé dans une huile destinée à être protégée de l'oxydation, par exemple une huile riche en acides gras insaturés, de préférence à raison de 1 à 2% en poids.

Dans un mode de réalisation préféré, le mélange antioxydant peut se présenter sous une forme plus fluide et contenir par exemple un triglycéride à chaîne moyenne.

Selon une mise en oeuvre particulièrement avantageuse du point de vue de l'activité antioxydante, le mélange contient en outre du tocophérol procurant une synergie. Comme tocophérol, on peut utiliser l'alpha-, le bêta-, le gamma-, le delta-tocophérol ou un mélange de ces tocophérols, par exemple un mélange naturel provenant de la fraction insaponifiable d'une huile végétale, par exemple de l'huile de soja, de l'huile de germes de blé, de l'huile de graines de coton. Le tocophérol peut être ajouté au mélange antioxydant ou se trouver naturellement présent dans l'huile destinée à être protégée.

Le mélange antioxydant contient avantageusement 1 à 5%, de préférence environ 2,5% en poids de tocophérol et 2,5 à 30%, de préférence 5 à 20% en poids de complexe, par exemple d'ascorbate de niacinamide.

Le mélange antioxydant peut servir de support à d'autres vitamines liposolubles, par exemple la vitamine A et constituer un micronutriment de grande valeur nutritionnelle lorsqu'il contient par exemple du MCT.

Dans le même ordre d'idées, il peut servir de support vitaminique destiné à être incorporé dans la phase lipidique lors de la préparation d'un aliment diététique tel que, par exemple un lait infantile et constituer alors un moyen particulièrement avantageux d'introduction des vitamines B et C, non liposolubles.

Le mélange antioxydant peut être incorporé dans un produit alimentaire contenant une matière grasse insaturée, particulièrement une huile végétale, par exemple de germes de blé, de pépins de cassis, de Kiwi, de maïs, de soja, de carthame, d'olive, d'onagre, de bourrache ou une matière grasse animale comme l'huile de beurre, la graisse de poule et particulièrement une huile de poisson.

Ce peut être par exemple une sauce à salade ou un produit diététique.

Le mélange antioxydant selon l'invention du fait de sa faible acidité, de pH ≥ 4, peut être aisément encapsulé, par exemple en gélules sans que l'eau résiduelle associée à la gélatine ne conduise au dégommage de la lécithine lors d'un entreposage prolongé.

Le mélange antioxydant peut être utilisé pour la protection des lipides contre l'oxydation dans les compositions destinées à l'alimentation entérale et parentérale.

Le mélange antioxydant peut également servir à la protection contre l'oxydation des lipides des compositions cosmétiques.

Les exemples ci-après illustrent l'invention. Dans ces exemples les parties et pourcentages sont pondéraux sauf indication contraire.

Exemple 1

On dissout 96,7 g d'ascorbate de niacinamide dans 5 l d'éthanol ayant été préalablement dégasé par sonication pour en éliminer l'oxygène libre, sous agitation modérée à 50°C sous un courant d'azote, puis on y ajoute 28,1 g de d,l-alpha-tocophérol. On pompe ensuite cette solution dans un flacon évaporateur rotatif de 25 l dans lequel on a préalablement introduit 1000 g de lécithine contenant 45% de phosphatidylcholine de soja dissout dans l'huile de carthame, à 50°C, ce qui conduit à une rapide dissolution de la lécithine, cette opération étant effectuée sous un courant d'azote. On obtient ainsi une solution translucide dépourvue de matières insolubles. On concentre ensuite la solution jusqu'à évaporation complète du solvant à 60°C sous un faible courant d'azote, puis après avoir refroidi la solution à la température ambiante, on en élimine toute trace de solvant résiduel par barbottage d'azote. Le mélange antioxydant liposoluble obtenu a une coloration jaune pâle et une viscosité semblable à celle d'une lécithine commerciale courante.

Il a la composition suivante:

|  | % |
|---|---|
| d,l-alpha-tocophérol | 2,5 |
| Vitamine C (sous forme d'ascorbate de niacinamide) | 5 |
| Vitamine B3 (sous forme d'ascorbate de niacinamide) | 3,6 |
| Phosphatidylcholine<br>Huile de carthame | 40<br>complément à 100 |

Exemple 2

Pour préparer un mélange antioxydant liposoluble plus fluide que celui de l'exemple 1, on procède comme à l'exemple 1 en ajoutant 2000 g d'une lécithine constituée de 45% de phosphatidylcholine, de 50% de triglycéride à chaîne moyenne (en C8-C10) et de 5% d'autres phosphatides.

Exemple 3

On prépare un mélange antioxydant liposoluble comme à l'exemple 1 sauf que la lécithine mise en oeuvre contient 45% de phosphatidylcholine, 50% de beurre de cacao et 5% d'autres phosphatides.

Exemple 4

On prépare un mélange antioxydant liposoluble comme à l'exemple 1, sauf que la lécithine mise en oeuvre contient 45% de phosphatidylcholine, 50% d'huile de soja et 5% d'autres phosphatides.

Exemple 5

On prépare un mélange antioxydant liposoluble comme à l'exemple 1, sauf que la lécithine mise en oeuvre contient 45% de phosphatidylcholine, 50% de triglycéride à chaîne moyenne (en C8-C10) et 5% d'autres phosphatides.

Exemple 6

On prépare un mélange antioxydant liposoluble comme à l'exemple 1, sauf que l'on n'y ajoute pas de d,l-alpha-tocophérol.

Exemples 7-9

On prépare les huiles spéciales ci-après par simple dissolution, sous faible agitation à 40-50°C sous azote, du mélange antioxydant selon l'exemple 1 mais ne contenant pas de d,l-alpha-tocophérol dans une huile, puis on ajoute à la solution du d,l-alpha-tocophérol dans les proportions indiquées au tableau 1 ci-après:

Tableau I

| Composition (g) | Exemple | | |
|---|---|---|---|
| | 7 | 8 | 9 |
| Huile de kiwi riche en acides gras hautement insaturés | --- | --- | 1000 |
| Huile de pépins de cassis | 1000 | --- | --- |
| Huile de poissons, riche en acide eicosapentaénoïque | --- | 1000 | --- |
| d,l-Alpha-tocophérol | 0,25 | 0,25 | 0,236 |
| Mélange antioxydant fournissant | 10,6 | 15,9 | 10 |
| ...g d'ascorbate de niacinamide | 0,5 | 0,75 | 0,472 |

Exemple 10

On prépare des capsules de gélatine, contenant 500 mg du mélange selon l'exemple 1 et les micronutriments indiqués dans les proportions indiquées. La préparation nutritive vitaminée a la composition indiquée ci-après:

| Composition | mg |
|---|---|
| Huile de pépins de cassis | 280 |
| Acétate de vitamine A (dissout dans l'huile de pépins de cassis) | 0,12 |
| Vitamine E sous forme de d,l-alpha-tocophérol (apporté par le mélange antioxydant selon l'exemple 1) | 1,33 |
| Tétrabutyrate de vitamine B2 (dissout dans l'huile de pépins de cassis) | 0,3 |
| Vitamine C (sous forme d'ascorbate de niacinamide et lécithine apporté par le mélange antioxydant selon l'exemple 1) | 2,661 |
| Vitamine B3 (sous forme d'ascorbate de niacinamide et lécithine apporté par le mélange antioxydant selon l'exemple 1) | 1,849 |
| Phosphatidylcholine de soja (sous forme de phosphatidylcholine dans l'huile de carthame) | 125 |
| Huile de carthame | 125 |

Les gélules sont stables à l'entreposage prolongé à la température ambiante.

Exemple 11

On prépare une huile destinée à assaisonner une salade de légumes frais sans vinaigre ayant la composition suivante.

```
Composition                                                    g
```

Huile de carthame raffinée contenant                          900
500 mg de d,l-alpha-tocophérol

Mélange antioxydant selon l'exemple 1                         100
apporter 5 g de vitamine C
            3,6 g de vitamine B3 et
          40 g de phosphatidylcholine

Une assiette de légumes frais de goût neutre peut être agrémentée avec 10 g du mélange précédent et apporter ainsi 50 mg de vitamine C, 36 mg de vitamine B3 et 400 mg de phosphatidylcholine.

Exemple 12

En utilisant le test d'oxydation accéléré Rancimat®, on détermine les temps d'induction de l'huile de pépins de cassis ou de l'huile de kiwi stabilisée avec les additifs antioxydants. Pour le test, on fait passer de l'air dans un tube de réaction contenant un échantillon de 5 g de matière grasse à 80°C et l'on mesure la conductivité des produits secondaires volatils formés en cours d'oxydation et entraînés avec le courant d'air. On détermine le temps d'induction graphiquement à partir de la courbe enregistrée de la conductivité en fonction du temps par intersection de la tangente à la courbe avec l'axe des temps.

Les résultats sont indiqués dans le tableau II ci-après.

Tableau II

| Huile | Additif antioxydant | Temps d'induction (h) |
|---|---|---|
| Huile de pépins de cassis | --- | 20 |
| Huile de pépins de cassis | Ascorbyl-palmitate (200 parties par million) | 40 |
| Huile de pépins de cassis | mélange antioxydant selon l'exemple 1 (1%) | 68,3 |
| Huile de pépins de cassis | mélange antioxydant selon l'exemple 1 (2%) | 78,3 |
| Huile de kiwi | --- | 5,75 |
| Huile de kiwi | Hydroxybutylanisole (600 parties par million) | 16,75 |
| Huile de kiwi | mélange antioxydant selon l'exemple 3 (1%) | 28,5 |
| Huile de kiwi | mélange antioxydant selon l'exemple 4 (1%) | 35,1 |
| Huile de kiwi | mélange antioxydant selon l'exemple 5 (1%) | 36,6 |

On constate que le mélange antioxydant selon l'invention augmente le temps d'induction de 3,4 à 3,9 fois par rapport à celui mesuré sans additif et a une activité antioxydante 1,7 à 2 fois plus importante que l'ascorbyl-palmitate dans le cas de l'huile de cassis.

Pour ce qui concerne l'huile de kiwi, le mélange contenant en sus du tocophérol augmente de 5 à 6 fois le temps d'induction par rapport à celui mesuré sans additif et a une activité antioxydante 1,7 à 2,2 fois plus importante que l'hydroxybutylanisole.

Exemples 13-16

Ces exemples concernent la préparation de compositions cosmétiques de soin de la peau contenant des lipides dont la partie lipidique est protégée contre l'oxydation par addition dans la phase lipidique du mélange oxydant liposoluble selon l'invention.

Dans ces exemples, la nomenclature utilisée est celle de la "Cosmetic, Toiletery and Flagrance Association, Inc. Washington DC" (CFTA).

Pour fabriquer les émulsions, on mélange les composants de la phase lipidique A et on la chauffe à 75°C. On prépare la phase aqueuse B et on la chauffe également à 75°C, puis on l'ajoute à la phase lipidique A en brassant lentement et on refroidit ensuite sous brassage lent jusqu'à la température ambiante, soit environ 25°C. A cette température, on ajoute lentement le cas échéant les constituants C dans l'ordre de la formule.

Pour fabriquer le produit anhydre de l'exemple 16, on mélange tous les constituants à froid, suivant l'ordre de la formule sous brassage lent.

## 13. Crème hydratante (émulsion huile-dans-l'eau)

| | % |
|---|---|

**Phase lipidique A**

| | |
|---|---|
| Peg-6-stéarate, glycerylstéarate et peg-20-cétyl éther (peg: polyéthylèneglycol) | 15 |
| Huile de vaseline | 5 |
| Huile de germes de blé contenant 1% du mélange antioxydant selon l'exemple 6 | 3 |
| Huiles d'amandes douces | 2 |
| Alcool cétylique | 1 |
| Isostéaryl-isostéarate | 2 |
| 2-Octyl-docécyl-myristate | 1 |
| Cire de lanoline | 1 |

**Phase aqueuse B**

| | |
|---|---|
| Méthylisothiazoline | 0,1 |
| Eau déminéralisée | 59,6 |
| Protéine de placenta humain | 2 |

**Additifs C**

| | |
|---|---|
| Propylèneglycol et extrait de calendula | 2 |
| Collagène soluble (2) dans l'eau déminéralisée (4) | 6 |
| Parfum | 0,3 |

7

## 14. Crème anti-rides (émulsion huile-dans-l'eau)

| | % |
|---|---|
| **Phase lipidique A** | |
| | |
| Cire d'abeille hydrophile non ionique | 10 |
| Huile de vaseline | 4 |
| Isostéaryl-isostéarate | 5 |
| Ethyl-linoléate | 1 |
| Huile minérale, huile de noyaux d'abricots et extrait de calendula | 4 |
| Huile d'amandes douces contenant 2% du mélange antioxydant selon l'exemple 6 | 3 |
| Huile de noyaux d'abricots | 3 |
| | |
| **Phase aqueuse B** | |
| | |
| Méthylisothiazole | 0,1 |
| Eau déminéralisée | 64,7 |
| Carbopol 934 (polymère d'acide acrylique polyréticulé) | 0,3 |
| Triéthanolamine | 0,6 |
| Propylèneglycol et extrait de sureau | 2 |
| Collagène hydrosoluble | 2 |
| Parfum | 0,3 |

15. <u>Crème pour bébés (émulsion huile-dans-l'eau)</u>

%
___

<u>Phase lipidique A</u>

| | % |
|---|---|
| Polyéthylèneglycol-6-32-stéarate | 10 |
| Huile d'amandes douces contenant 1% de mélange antioxydant selon l'exemple 6 | 6 |
| Huile de vaseline | 4 |
| Huile de palmiste, huile de palme et peg 6 | 3 |
| Acide stéarique | 1 |

<u>Phase aqueuse B</u>

| | |
|---|---|
| Eau déminéralisée | 67,5 |
| Glycérine | 3 |
| Méthylisothiazoline | 1 |
| Propylèneglycol et extrait de calendula | 4 |
| Parfum | 0,5 |

16. <u>Huile satinée (anhydre)</u>

| | |
|---|---|
| Huile de tournesol contenant 1% du mélange antioxydant selon l'exemple 6 | 3 |
| Triglydérides d'acides caprylique et caprique | 30 |
| Diméthyl polysiloxane cyclique | 25,7 |
| Propylèneglycol-dipélargonate | 37,8 |
| Octyl méthoxycinnamate | 3 |
| Parfum | 0,5 |

**Revendications**

1. Mélange antioxydant liposoluble à base d'acide organique, caractérisé par le fait qu'il est constitué d'un complexe de vitamine du groupe B et d'acide organique et d'une lécithine contenant au moins 40% en poids de phosphatidylcholine.

2. Mélange selon la revendication 1, caractérisé par le fait que le complexe est l'ascorbate de niacinamide ou de pyridoxyne.

3. Mélange selon la revendication 1, caractérisé par le fait qu'il contient en plus du tocophérol.

**4.** Mélange selon la revendication 2 ou 3, caractérisé par le fait qu'il contient un triglycéride à chaîne moyenne.

**5.** Micronutriment, caractérisé par le fait qu'il contient un mélange selon l'une des revendications 1 à 4 et des vitamines sous forme liposoluble.

**6.** Huile riche en acides gras insaturés protégée contre l'oxydation, caractérisée par le fait qu'elle contient un mélange selon l'une des revendications 1 à 4.

**7.** Produit alimentaire protégé contre l'oxydation, caractérisé par le fait qu'il contient un mélange selon l'une des revendications 1 à 4.

**8.** Produit cosmétique protégé contre l'oxydation, caractérisé par le fait qu'il contient un mélange selon l'une des revendications 1 à 4.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 326 829  (SOCIETE DES PRODUITS NESTLE) <br> * page 2, lignes 4-10; page 3, ligne 3 * <br> --- | 1-8 | A 61 K    7/00 <br> A 23 D    9/06 <br> C 11 B    5/00 |
| Y | EP-A-0 353 161  (L'OREAL) <br> * page 2, lignes 17-34 * <br> --- | 1-8 | |
| Y | US-A-2 432 698  (A. TAUB et al.) <br> * colonne 3, ligne 74 - colonne 4, ligne 2; colonne 4, table I * <br> --- | 1-8 | |
| Y | REVUE FRANCAISE DES CORPS GRAS vol. 34, nos. 5,6, mai/juin 1987, pages 271-274, Paris, FR; J. CILLARD et al.: "Antioxidant activity of associated alpha-tocopherol and ascorbic acid in aqueous media" <br> * page 274, colonne 1, alinéas 2-5 * <br> --- | 1-8 | |
| A | GB-A- 902 377  (U.S. VITAMINS & PHARMACEUTICAL CORPORATION) <br> * colonne 1, lignes 19-24 * <br> ----- | 1-8 | **DOMAINES TECHNIQUES RECHERCHES (Int. Cl.5)** <br><br> A 61 K <br> A 23 D <br> C 11 B |

Le présent rapport a été établi pour toutes les revendications

| Lieu de la recherche | Date d'achèvement de la recherche | Examinateur |
|---|---|---|
| BERLIN | 16-12-1991 | AVEDIKIAN P.F. |

EPO FORM 1503 03.82 (P0402)